# EUROPEAN PATENT APPLICATION

(11) **EP 4 056 220 A1**
(43) Date of publication of application: **14.09.2022**
(21) Application number: 20884292.2
(22) Date of filing: 04.11.2020
(51) Int. Cl.: A61M 25/00

(54) **CATHETER**

(30) Priority: 08.11.2019 WO PCT/JP2019/043835
(71) Applicant: ASAHI INTECC CO., LTD., Seto-shi, Aichi 489-0071 (JP)
(72) Inventor: KUBOKI Teppei, Seto-shi, Aichi 489-0071 (JP); FUJINO Keiichi, Seto-shi, Aichi 489-0071 (JP)
(74) Representative: Winter, Brandl - Partnerschaft mbB
(86) International application number: PCT/JP2020/041146
(87) International publication number: WO 2021/090821

(57) **Abstract**

The catheter includes a coil body, a braided body, an inner resin layer, a cushioning material, and an outer resin layer. The braided body is formed into a tubular shape by braiding a plurality of wires, and has an intracoil braided portion arranged on the inner peripheral side of the coil body and an extracoil braided portion arranged so as to extend beyond the coil body toward the distal end. The inner resin layer coats the inner peripheral surface of the braided body. The cushioning material is in contact with a part closer to the coil body in the extracoil braided portion of the braided body, as well as in contact with the distal end of the coil body. The outer resin layer coats the outer peripheral surface of the extracoil braided portion of the braided body, the cushioning material, and the coil body.

## Description

### TECHNICAL FIELD

The technique disclosed herein relates to a catheter.

### BACKGROUND ART

Several catheter-based methods have been widely employed to treat or examine a constricted part or an occluded part in a blood vessel etc., or an abnormal blood vessel (hereinafter referred to as "lesion"). Previously, a catheter has been known which includes a first braid and a second braid embedded in a tubular resin layer (see, e.g., Patent Literature 1). Both of the first braid and the second braid are braided bodies formed into tubular shapes by braiding a plurality of wires. The first braid is located continuously from the distal end to the proximal end of the catheter. The second braid is placed on the catheter continuously from the intermediate position to the proximal end and not in the vicinity of the distal end of the catheter. Thus, the vicinity of the distal end of the catheter has relatively high flexibility, and the proximal end of the catheter has relatively high rigidity.

### CITATION LIST

### Patent Literature

Patent Literature 1: JP 2019-37572 A

### SUMMARY OF INVENTION

### TECHNICAL PROBLEM

The conventional catheter described above has a problem in that there is a large difference in rigidity between the vicinity of the distal end, where the second braid is not placed, and the vicinity of the proximal end, where the second braid is placed, thus facilitating occurrence of a kink, where the boundary site between the vicinity of the distal end and the vicinity of the proximal end of the catheter is bent and no longer reverts to its original position due to the difference in rigidity.

Such problem should be solved not only for catheters that are guided into a lesion in a blood vessel or the like, using a guide wire, but also especially for catheters that are inserted into a lesion in a blood vessel or the like, without use of a guide wire, as proposed by the inventor.

Disclosed herein is a technology that provides solution to the problem described above.

### SOLUTION TO PROBLEM

A technology disclosed herein can be achieved as e.g., the following aspects.
(1) The catheter disclosed herein is a catheter having a coil body; a braided body formed into a tubular shape by braiding a plurality of wires, the braided body having an intracoil braided portion arranged on the inner peripheral side of the coil body and an extracoil braided portion arranged so as to extend beyond the coil body toward the distal end; an inner resin layer coating the inner peripheral surface of the braided body; a cushioning material in contact with a part closer to the coil body in the extracoil braided portion of the braided body as well as in contact with the distal end of the coil body, the cushioning material having rigidity between the rigidity of the braided body and the rigidity of the coil body; and an outer resin layer coating the outer peripheral surface of the extracoil braided portion of the braided body, the cushioning material, and the coil body.
   In this catheter, the coil body is not placed in the vicinity of the distal end of the catheter (where the extracoil braided portion of the braided body is located), and the coil body is placed in the vicinity of the proximal end of the catheter (where the intracoil braided portion of the braided body is located). Thus, the vicinity of the distal end of the catheter has relatively high flexibility and the proximal end of the catheter has relatively high rigidity. This can provide improved performance of the distal end of the catheter to select a smaller-diameter blood vessel, and improved torquability (rotational performance) from the proximal end side to the distal end side of the catheter. In addition, the catheter includes a cushioning material. The cushioning material coats a part closer to the coil body in the extracoil braided portion of the braided body, as well as is in contact with the distal end of the coil body. The cushioning material also has rigidity between the rigidity of the braided body and the rigidity of the coil body. This reduces a difference in rigidity between the vicinity of the distal end, where no coil body is placed, and the vicinity of the proximal end, where a coil body is placed, and consequently allows, for example, ensuring torquability of the catheter, as well as preventing occurrence of a kink, where the boundary site between the vicinity of the distal end and the vicinity of the proximal end of the catheter is bent and no longer reverts to its original position due to the difference in rigidity.
(2) In the catheter described above, the extracoil braided portion of the braided body may be configured to have a reduced diameter part having an outer diameter reduced toward the distal end of the catheter, and a constant diameter part adjacent to the distal end of the reduced diameter part and having the same outer diameter throughout the entire length in the axial direction of the catheter. The catheter can prevent an extracoil braided portion from being easily pulled out from a blood vessel after insertion into the blood vessel, compared to a configuration including an extracoil braided portion having an outer diameter becoming smaller toward the distal end throughout the entire length.
(3) In the catheter described above, the catheter may be configured such that the inner diameter of the inner resin layer in at least a part of the extracoil braided portion is smaller than the inner diameter of the inner resin layer in the intracoil braided portion. The catheter includes a part having a relatively large inner diameter in the vicinity of the proximal end of the catheter, thus allows ensuring a large volume of accommodation space inside the catheter, and for example, accommodating a large amount of fluid (e.g., contrast medium) in the vicinity of the proximal end of the catheter, when the fluid is injected from the distal end of the catheter. In addition, since the vicinity of the distal end of the catheter includes a part with a relatively small inner diameter, the flow velocity of the fluid delivered from the proximal end side of the catheter can be increased at the distal end side to improve the jet force of the fluid from the catheter.
(4) In the catheter described above, the configuration may be such that the radial length of the catheter in the vicinity of the distal end of the cushioning material is smaller than the radial length in the vicinity of the proximal end of the cushioning material. This catheter further reduces a difference in rigidity between the vicinity of the distal end and the vicinity of the proximal end of the catheter, thereby allowing more effective prevention of occurrence of a kink, compared to a configuration having a cushioning material with a uniform radial length.
(5) In the catheter described above, the cushioning material may be configured to have an extended portion that extends so as to lie over a plurality of wires that composes the coil body as well as are adjacent to each other. In this catheter, the cushioning material has the extended portion, which causes high adhesion between the cushioning material and the coil body. This further reduces a difference in rigidity between the vicinity of the distal end and the vicinity of the proximal end of the catheter, thereby allowing more effective prevention of occurrence of a kink.
(6) In the catheter described above, the configuration may have a medium resin layer that is filled in gaps in the braided body and disposed between the outer resin layer and inner resin layer. In the catheter, the medium resin layer allows preventing disruption of the braided body and improving adhesiveness between the outer resin layer and the inner resin layer.
(7) The catheter disclosed herein includes a coil body; a braided body formed into a tubular shape by braiding a plurality of wires, the braided body having an intracoil braided portion arranged on the inner peripheral side of the coil body and an extracoil braided portion arranged so as to extend beyond the coil body toward the distal end; an inner resin layer coating the inner peripheral surface of the braided body; and an outer resin layer coating the outer peripheral surface of the extracoil braided portion of the braided body and the coil body. The catheter has a catheter distal end part that includes the extracoil braided portion, a catheter proximal end part that includes the coil body and the intracoil braided portion, and a catheter connection part that connects the catheter distal end part and the catheter proximal end part in the axial direction of the catheter. The catheter distal end part has a flexural rigidity of 0.005 gf·cm²/cm or more to less than 0.05 gf·cm²/cm; the catheter connection part has a flexural rigidity of 0.05 gf·cm²/cm or more to less than 1.4 gf·cm²/cm; and the catheter proximal end part has a flexural rigidity of 1.4 gf·cm²/cm or more to 3.0 gf·cm²/cm or less.
   In the catheter, the coil body is not placed in the vicinity of the distal end of the catheter (a catheter distal end part, where the extracoil braided portion of the braided body is located), and the coil body is placed in the vicinity of the proximal end of the catheter (a catheter proximal end part, where the intracoil braided portion of the braided body is located). Thus, the vicinity of the catheter distal end part has relatively high flexibility, and the catheter proximal end part has relatively high rigidity. This can provide improved performance of the catheter distal end part to select a smaller-diameter blood vessel, and improved torquability (rotational performance) from the catheter proximal end part to the catheter distal end part. In addition, the catheter has a catheter connection part that connects the catheter distal end part to the catheter proximal end part in the axial direction of the catheter. The catheter connection part has rigidity between the rigidity of the catheter distal end part and the rigidity of the catheter proximal end part. This reduces a difference in rigidity between the catheter distal end part, where no coil body is placed, and the catheter proximal end part, where a coil body is placed, and consequently allows, for example, ensuring torquability of the catheter, as well as preventing occurrence of a kink, where the boundary site between the vicinity of the distal end and the vicinity of the proximal end of the catheter is bent and no longer reverts to its original position due to the difference in rigidity. Meanwhile, there is a conventional technique in which a guiding catheter is inserted into a blood vessel, and then a guide wire and a microcatheter are inserted into the guiding catheter and advanced alternately. In this regard, when a blood vessel is relatively straight from the site of catheter insertion to the site to be treated, use of the catheter and a guide wire allows execution of the technique described above with no need for any guiding catheter.
(8) In the catheter described above, the configuration may be such that a part of the coil body is placed in the catheter connection part, and that the wire composing the part of the coil body becomes thinner toward the distal end of the catheter connection part. The catheter allows reducing a difference in rigidity between the catheter proximal end part and the catheter connection part and ensuring torquability of the catheter, as well as preventing occurrence of a kink of the catheter due to the difference in rigidity between the catheter distal end part and the catheter proximal end part.
(9) In the catheter described above, the configuration may be such that a part of the extracoil braided portion is placed in the catheter connection part, and that the wire composing the part of the extracoil braided portion has a diameter increasing toward the distal end of the coil body. The catheter allows reducing a difference in rigidity between the catheter distal end part and the catheter connection part and ensuring torquability of the catheter, as well as preventing occurrence of a kink of the catheter due to the difference in rigidity between the catheter distal end part and the catheter proximal end part.
(10) In the catheter described above, the configuration may be such that the outer diameter of the catheter is the same throughout the entire length. In the configuration including the same outer diameter of the catheter throughout the entire length, the catheter allows ensuring torquability of the catheter, as well as preventing occurrence of a kink of the catheter due to a difference in rigidity between the catheter distal end part and the catheter proximal end part.

The technology disclosed herein can be achieved in various forms, e.g. in a form of a catheter, a manufacturing method of a catheter, and the like.

### BRIEF DESCRIPTION OF DRAWINGS

Fig. 1 is an explanatory diagram schematically illustrating a longitudinal section configuration of a catheter in a first embodiment.
Fig. 2 is an enlarged view of a longitudinal section configuration of a part of a catheter body of the catheter.
Fig. 3 is an enlarged view of a part of the braided body included in the catheter.
Fig. 4 is a transverse sectional view of the catheter body at the position IV-IV in Fig. 2.
Fig. 5 is a transverse sectional view of the catheter body at the position V-V in Fig. 2.
Fig. 6 is a transverse sectional view of the catheter body at the position VI-VI in Fig. 2.
Fig. 7 is an explanatory diagram illustrating a longitudinal section configuration of a part of a catheter in a second embodiment.
Fig. 8 is an explanatory diagram illustrating a longitudinal section configuration of a part of a catheter in a third embodiment.
Fig. 9 is an explanatory diagram illustrating a longitudinal section configuration of a part of a catheter in a fourth embodiment.
Fig. 10 is a transverse sectional view of the catheter body at the position X-X in Fig. 9.
Fig. 11 is an explanatory diagram schematically illustrating a longitudinal section configuration of a catheter in a fifth embodiment.
Fig. 12 is an explanatory diagram illustrating a longitudinal section configuration of a part of a catheter.
Fig. 13 is a graph showing the overall flexural rigidity of catheters.
Fig. 14 is a graph showing flexural rigidity of a catheter distal end part and a catheter connection part in catheters.
Fig. 15 is an explanatory diagram illustrating a longitudinal section configuration of a part of a catheter in a sixth embodiment.
Fig. 16 is an explanatory diagram illustrating a longitudinal section configuration of a part of a catheter in a seventh embodiment.

### EMBODIMENTS OF THE INVENTION

### A. First Embodiment:

### A-1. Overall Configuration of Catheter 100:

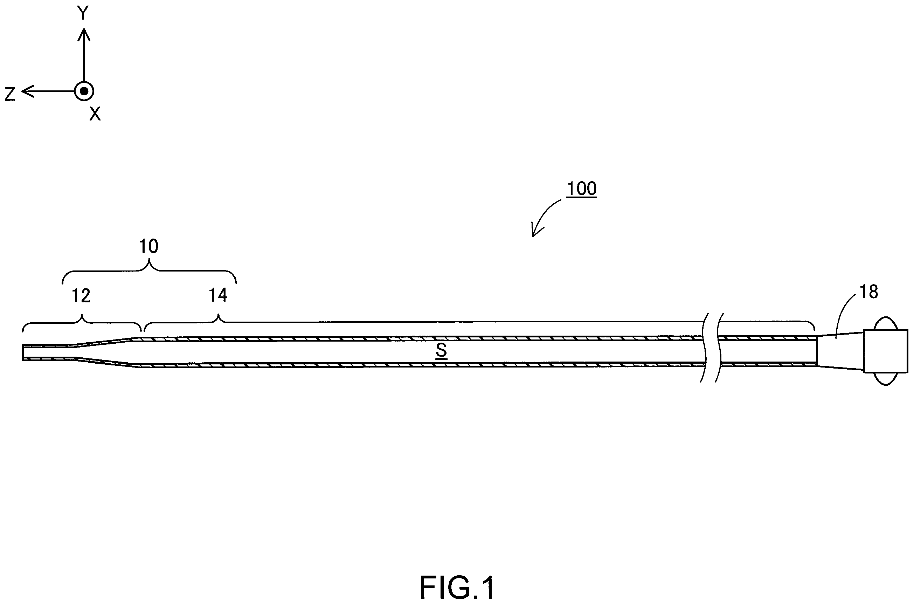

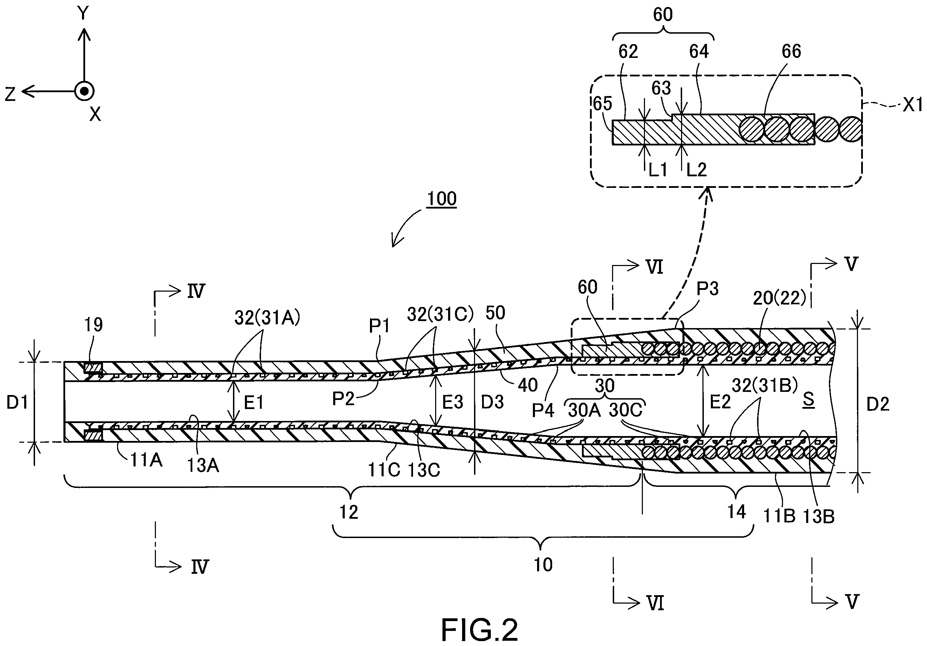

Fig. 1 is an explanatory diagram schematically illustrating a longitudinal section configuration of a catheter 100 in a first embodiment. Note that Fig. 1 omits the detailed configuration of a catheter body 10, described below. Fig. 2 is an explanatory diagram of a longitudinal section configuration of a part of the catheter body 10 of the catheter 100 in an enlarged view. Fig. 2 further shows a configuration of a part X1 in the catheter 100 (a cushioning material 60 described later) in an enlarged view. The longitudinal section of the catheter 100 refers to a cross section (a XY cross section in Fig. 1) parallel to the axial direction of the catheter 100 (longitudinal direction, the Z-axial direction in Fig. 1 and Fig. 2), and the transverse section of the catheter 100 refers to a cross section (a YZ cross section in Fig. 1) perpendicular to the axial direction of the catheter 100. In Fig. 1, the reverse direction of the Z-axis (closer to a connector 18 described later) represents a proximal end side (near side) to be manipulated by a technician such as a physician, and the forward direction of the Z-axis (opposite to the connector 18) represents a distal end side (far side) to be inserted into the body. Although Fig. 1 and Fig. 2 shows the catheter 100 in an entirely straight form parallel to the direction of the Z-axis, the catheter 100 has enough flexibility to be bent.

The catheter 100 is a medical device to be inserted into a blood vessel or the like to treat or examine a lesion in the blood vessel or the like. As shown in FIG. 1, the catheter 100 includes a catheter body 10, and the connector 18 attached to the proximal end of the catheter body 10.

The catheter body 10 has a distal end part 12 and a proximal end part 14. The distal end part 12 is a part that includes the distal end of the catheter body 10 and has relatively high flexibility due to absence of a coil body 20 described later. The proximal end part 14 is a part that includes the proximal end of the catheter body 10 and has relatively high rigidity due to presence of the coil body 20. Specific description will be provided below.

As shown in Figs. 1 and 2, the catheter body 10 is a tubular (e.g., cylindrical) member with openings at the distal end and the proximal end. As used herein, "tubular (cylindrical)" is not limited to a completely tubular (cylindrical) shape, but may also be an overall approximately tubular shape (an approximately cylindrical shape such as a slightly conical shape or a partially uneven shape). Inside the catheter body 10, a lumen S is formed with extending from the distal end to the proximal end of the catheter body 10. The lumen S, for example, receives supply of fluid such as an embolic substance to be injected into the abnormal vessel, or insertion of a guide wire, which is not depicted.

As shown in Fig. 2, the catheter body 10 has a distal end-side constant diameter part 11A, a proximal end-side constant diameter part 11B, and a connection part 11C. The distal end-side constant diameter part 11A is a part that includes the distal end of the catheter body 10, and a first outer diameter D1 of the distal end-side constant diameter part 11A is approximately the same throughout the entire length in the axial direction of the catheter 100. The proximal end-side constant diameter part 11B is a part that includes the proximal end of the catheter body 10, and a second outer diameter D2 of the proximal end-side constant diameter part 11B is larger than the first outer diameter D1 of the distal end-side constant diameter part 11A and approximately the same throughout the entire length in the axial direction of the catheter 100. The connection part 11C is a part that is located between the distal end-side constant diameter part 11A and the proximal end-side constant diameter part 11B, and connects the distal end-side constant diameter part 11A to the proximal end-side constant diameter part 11B, and the third outer diameter D3 of the connection part 11C continuously increases from the distal end side toward the proximal end side. In the embodiment, the distal end-side constant diameter part 11A and the vicinity of the distal end of the connection part 11C compose the distal end part 12 described above, and the vicinity of the proximal end of the connection part 11C and the proximal end-side constant diameter part 11B compose the proximal end part 14 described above.

A first inner diameter E1 of a first inner peripheral surface 13A in the vicinity of the distal end of the catheter body 10 is approximately the same throughout the entire length in the axial direction of the catheter 100 (Z-axial direction) in the first inner peripheral surface 13A. A second inner diameter E2 of a second inner peripheral surface 13B in the vicinity of the proximal end of the catheter body 10 is larger than the first inner diameter E1 of the first inner peripheral surface 13A, and approximately the same throughout the entire length in the axial direction of the catheter 100 in the second inner peripheral surface 13B. A third inner peripheral surface 13C, which is located between the first inner peripheral surface 13A and the second inner peripheral surface 13B, continuously increases from the distal end side toward the proximal end side.

In the embodiment, as shown in Fig. 2, a boundary position P1 between the distal end-side constant diameter part 11A and the connection part 11C, and a boundary position P2 between the first inner peripheral surface 13A and the third inner peripheral surface 13C are approximately the same in the axial direction of the catheter 100 (Z-axial direction). By contrast, a boundary position P3 between the proximal end-side constant diameter part 11B and the connection part 11C is located closer to the proximal end than a boundary position P4 between the second inner peripheral surface 13B and the third inner peripheral surface 13C.

### A-2. Detailed Configuration of Catheter Body 10:

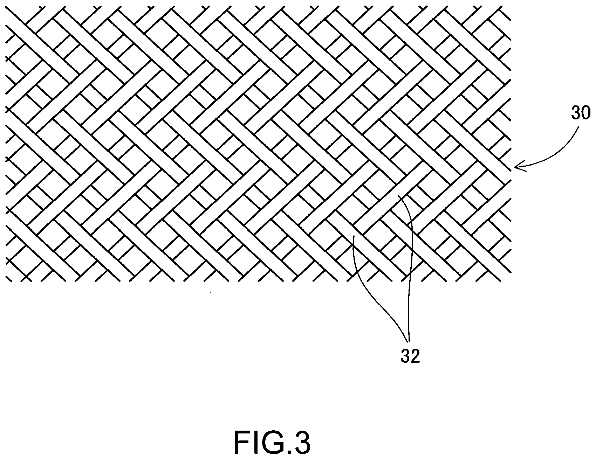

As shown in Fig. 2, the catheter body 10 includes the coil body 20, a braided body 30, an inner resin layer 40, an outer resin layer 50, and a cushioning material 60. Fig. 3 is an enlarged view of a part of the braided body 30.

The coil body 20 is a coiled member formed into a hollow cylindrical shape by spirally winding a plurality of wires 22. The coil body 20 is not placed in the distal end part 12 in the catheter body 10, but at the proximal end part 14. Particularly, the coil body 20 is continuously placed from the proximal end to the middle site (near the connection part 11C described above) in the catheter body 10, and is not placed in the vicinity of the distal end of the catheter body 10.

The distal end of the coil body 20 is located closer to the distal end of the catheter 100 than the boundary position P3 between the proximal end-side constant diameter part 11B and the connection part 11C, in the axial direction of the catheter 100 (Z-axial direction). This more effectively improves torquability from the proximal end part 14 to the distal end part 12 of the catheter 100. The distal end of the coil body 20 is also located closer to the proximal end than the boundary position P4 between the second inner peripheral surface 13B and the third inner peripheral surface 13C. This prevents increase in the outer diameters of the distal end-side constant diameter part 11A, the connection part 11C, and the like due to presence of the coil body 20.

The wire 22 may employ a metallic material. Examples of the metallic materials to be used can include radiopaque alloy such as stainless steel (SUS302, SUS304, SUS316, etc.), superelastic alloy such as Ni-Ti alloy, a piano wire, nickel-chromium-based alloy, or cobalt alloy; and radiopaque alloy such as gold, platinum, tungsten, or alloy containing these elements (e.g., platinum-nickel alloy). The outer diameter of the wire 22 is preferably larger than the outer diameter of a wire 32, which composes the braided body 30 described next. Furthermore, the rigidity of the wire 22 is preferably larger than the rigidity of the wire 32.

The braided body 30 is a tubular member derived by braiding a plurality of wires 32 braided. Particularly, as shown in Fig. 3, the braided body 30 has a mesh-like structure in which a plurality of wires 32 is braided so as to intersect each other. As shown in Fig. 2, the braided body 30 is continuously placed from the proximal end to the distal end in the catheter body 10. The braided body 30 has approximately the same thickness throughout the entire length in the axial direction of the catheter 100. As used herein, the thickness of each member refers to the radial length of the catheter 100.

The braided body 30 has an extracoil braided portion 30A and an intracoil braided portion 30B. The intracoil braided portion 30B is placed on the inner peripheral side of the coil body 20. The extracoil braided portion 30A is placed so as to extend from the intracoil braided portion 30B toward the distal end beyond the coil body 20. In other words, the coil body 20 is not placed on the outer periphery of the extracoil braided portion 30A in the braided body 30, but is placed so as to surround the outer periphery of the intracoil braided portion 30B.

The braided body 30 is shaped along the inner peripheral surface of the catheter body 10 (the first inner peripheral surface 13A, the second inner peripheral surface 13B, the third inner peripheral surface 13C). Particularly, in the braided body 30, a part located on the outer periphery of the first inner peripheral surface 13A corresponds to a first constant diameter part 31A, which has approximately the same outer diameter throughout the entire length in the axial direction of the catheter 100. In the braided body 30, a part located on the outer periphery of the second inner peripheral surface 13B corresponds to a second constant diameter part 31B, which has an outer diameter that is larger than the outer diameter of the first constant diameter part 31A and approximately the same throughout the entire length in the axial direction of the catheter 100. In the braided body 30, a part located on the outer periphery of the third inner peripheral surface 13C corresponds to a reduced diameter part 31C, which has an outer diameter continuously reduced toward the distal end. In the embodiment, the first constant diameter part 31A, the reduced diameter part 31C, and the distal end side of the second constant diameter part 31B compose the extracoil braided portion 30A, as well as are included in the distal end part 12, while the proximal end side of the second constant diameter part 31B composes the intracoil braided portion 30B, as well as is included in the proximal end part 14.

The wire 32 can employ a metallic material. Examples of the metallic materials to be used can include tungsten and stainless steel (SUS 302, SUS 304, SUS 316, etc.)

The inner resin layer 40 coats the inner peripheral surface of the braided body 30. Particularly, the inner resin layer 40 is continuously placed from the proximal end to the distal end of the catheter body 10, and coats the inner peripheral surface side of the braided body 30 throughout the entire length. In other words, the inner resin layer 40 composes the inner peripheral surface of the catheter body 10 (the inner peripheral surfaces 13A, 13B, and 13C as described above). The thickness of the inner resin layer 40 is approximately the same throughout the entire length in the axial direction of the catheter 100.

In the catheter body 10, the inner diameter of the inner resin layer 40 in at least a part of the extracoil braided portion 30A (the first inner diameter E1 of the first inner peripheral surface 13A in the vicinity of the distal end) is smaller than the inner diameter of the inner resin layer 40 in the intracoil braided portion 30B (the second inner diameter E2 of the second inner peripheral surface 13B in the vicinity of the proximal end). The inner resin layer 40 is also in close contact with the inner peripheral surface of the braided body 30, as well as enter each gap between the wires 32 composing the braided body 30. The inner resin layer 40 is composed of e.g., polytetrafluoroethylene (PTFE) resin.

The cushioning material 60 is in contact with a part closer to the coil body 20 in the extracoil braided portion 30A of the braided body 30, as well as in contact with the distal end of the coil body 20. In the embodiment, the shape of the cushioning material 60 is annular in the axial view of the catheter 100 (Z-axial direction), and is placed so as to surround the outer periphery of a part closer to the coil body 20 in the extracoil braided portion 30A. Particularly, the cushioning material 60 is placed so as to surround the outer periphery of the second constant diameter part 31B in the extracoil braided portion 30A and to make the inner peripheral surface of the cushioning material 60 in contact with the outer peripheral surface of the second constant diameter part 31B. This results in high adhesion between the cushioning material 60 and the braided body 30, and thus can provide improved effect for preventing a difference in rigidity between the distal end part 12 and the proximal end part 14 due to the cushioning material 60.

The thickness of the vicinity of the distal end of the cushioning material 60 is thinner than the thickness of the vicinity of the proximal end of the cushioning material 60. Particularly, the cushioning material 60 has a thinner part 62, which has a first thickness L1, and a thicker part 64, which is adjacent to the proximal end of the thinner part 62 and has a second thickness L2 thicker than the first thickness L1. Moreover, the inner peripheral surface of the cushioning material 60 has an approximately the same inner diameter throughout the entire length of the cushioning material 60, and the outer peripheral surface of the cushioning material 60 has a smaller outer diameter in the vicinity of the distal end (thinner part 62) than the outer diameter in the vicinity of the proximal end (thicker part 64). This forms a step face 63, which is directed to the distal end side of the catheter 100, on the outer peripheral surface of the cushioning material 60. The distal end face 65 of the cushioning material 60 is also directed to the distal end of the catheter 100. The cushioning material 60 has at least one of the step face 63 and the distal end face 65, thereby allowing prevention of separation between the cushioning material 60 and the coil body 20. That is, for example, in a process of insertion of the catheter body 10 into the body, the step face 63 or the distal end face 65 of the cushioning material 60 receives push pressure from the outer resin layer 50, thereby causing the cushioning material 60 to be pressed against the distal end of the coil body 20, and thus allowing prevention of separation between the cushioning material 60 and the coil body 20.

The cushioning material 60 (flexural rigidity, torsional rigidity) has rigidity between the rigidity of the braided body 30 and the rigidity of the coil body 20. In this embodiment, the rigidity of the cushioning material 60 is higher than the rigidity of the braided body 30 and lower than the rigidity of the coil body 20. Note that the magnitude relation between the rigidity of the cushioning material 60, the braided body 30, and the coil body 20 can be identified by, e.g., the composition and processing degree of each member. The cushioning material 60 can employ a polyolefin resin such as a polyamide resin, a polyamide elastomer, polyester, polyurethane, a polyvinyl chloride resin, polyethylene, polypropylene, or an ethylene-propylene copolymer; a fluorine resin such as Teflon^{®}; a resin such as various synthetic resin materials such as an ethylene-vinyl acetate copolymer; or a combination thereof. In the embodiment, for example, a polyamide elastomer is used.

The cushioning material 60 is placed between the boundary position P3 between the proximal end-side constant diameter part 11B and the connection part 11C, and the boundary position P4 between the second inner peripheral surface 13B and the third inner peripheral surface 13C, in the axial direction of the catheter 100 (Z-axial direction). The cushioning material 60 is thus placed in a relatively thick part in the catheter body 10, thus preventing the outer resin layer 50 around the cushioning material 60 from being thinner.

As shown in the X1 part in Fig. 2, the cushioning material 60 has an extended portion 66 that extends so as to lie over a plurality of wires 22 that composes the coil body 20 as well as are adjacent to each other. In the embodiment, the cushioning material 60 lies over the plurality of wires 22 adjacent to each other as well as enters the recesses formed between the plurality of wires 22. This results in a higher adhesion between the cushioning material 60 and the coil body 20. The extended portion 66 is also formed over the entire circumference of the coil body 20. The cushioning material 60 having the extended portion 66 as described above can be formed by, e.g., fitting a ring-shaped resin material around the periphery of the braided body 30 and performing heat treatment.

The outer resin layer 50 coats the outer peripheral surface of the extracoil braided portion 30A of the braided body 30, the cushioning material 60, and the coil body 20. Particularly, the outer resin layer 50 is continuously placed from the proximal end to the distal end of the catheter body 10, and coats the outer peripheral surface side of the extracoil braided portion 30A, the cushioning material 60, and the coil body 20 throughout the entire length. In other words, the outer resin layer 50 composes the outer peripheral surface of the catheter body 10. The outer resin layer 50 has an approximately the same thickness throughout the entire length in the axial direction of the catheter 100. The outer resin layer 50 is composed of e.g., polytetrafluoroethylene (PTFE) resin. The inner resin layer 40 and the outer resin layer 50 may be composed of the same type of resin as each other, or different types of resin from each other. The distal end part in the outer resin layer 50 has an annular chip 19 embedded therein.

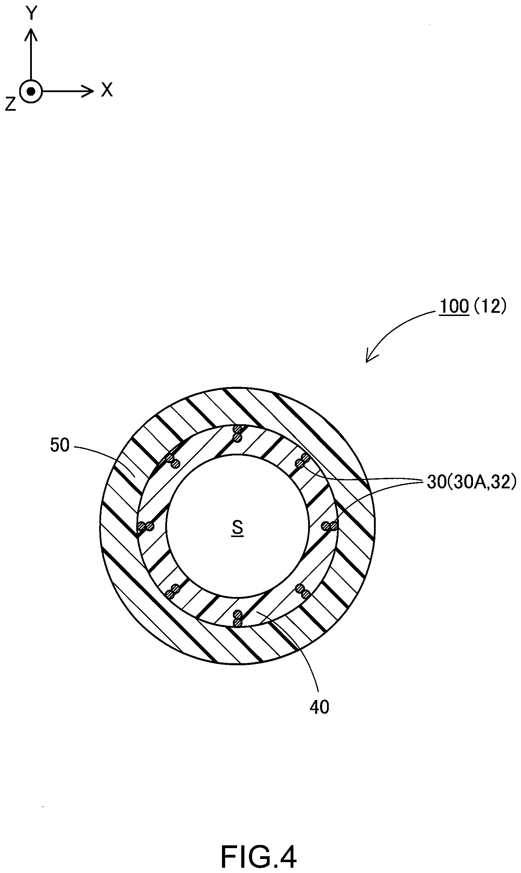

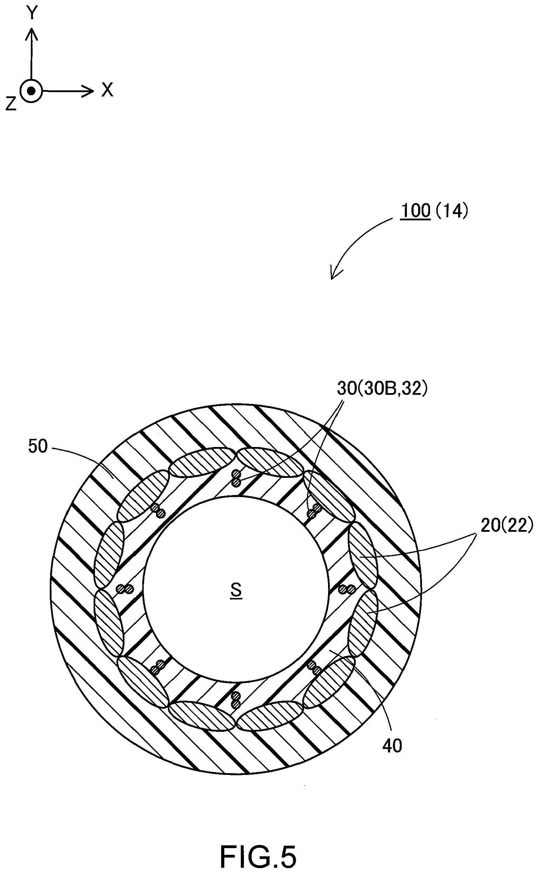

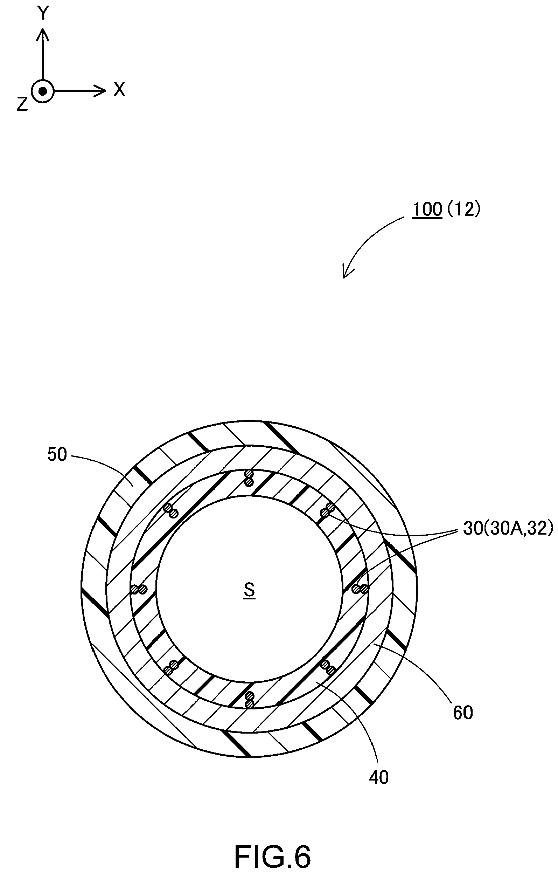

Fig. 4 is a transverse sectional view of the catheter body 10 at the position IV-IV in Fig. 2; Fig. 5 is a transverse sectional view of the catheter body 10 at the position V-V in Fig. 2; and Fig. 6 is a transverse sectional view of the catheter body 10 at the position VI-VI in Fig. 2. According to the configuration described above, as shown in Fig. 4, the distal end-side constant diameter part 11A in the catheter body 10 includes the braided body 30 (extracoil braided portion 30A), the inner resin layer 40, and the outer resin layer 50, but not the coil body 20. As shown in Fig. 5, the proximal end-side constant diameter part 11B in the catheter body 10 includes the coil body 20 in addition to the braided body 30 (intracoil braided portion 30B), the inner resin layer 40, and the outer resin layer 50. As shown in Fig. 6, a part of the connection part 11C in the catheter body 10 includes the braided body 30 (extracoil braided portion 30A), the inner resin layer 40, and the outer resin layer 50, and does not include the coil body 20, but further includes the cushioning material 60.

### A-3. Effect of the Embodiment:

As shown in Fig. 2, in the catheter 100 according to the embodiment, the coil body 20 is not placed in the vicinity of the distal end of the catheter 100 (the distal end part 12, where the extracoil braided portion 30A of the braided body 30 is located), and the coil body 20 is placed in the vicinity of the proximal end of the catheter (the proximal end part 14, where the intracoil braided portion 30B of the braided body 30 is located). Thus, the vicinity of the distal end part 12 of the catheter 100 has relatively high flexibility, and the proximal end part 14 of the catheter 100 has relatively high rigidity. This can provide improved performance of the distal end part 12 of the catheter 100 to select a smaller-diameter blood vessel (performance capable of accurately selecting each of a plurality of smaller-diameter blood vessels extending like branches and leaves from a thick main vessel), and improved torquability (rotational performance, butt rigidity) from the proximal end part 14 to the distal end part 12 of the catheter 100.

Furthermore, as shown in Fig. 2 and Fig. 6, the catheter 100 according to the embodiment includes the cushioning material 60. The cushioning material 60 is in contact with a part closer to the coil body 20 in the extracoil braided portion 30A of the braided body 30, as well as in contact with the distal end of the coil body 20. The cushioning material 60 also has rigidity between the rigidity of the braided body 30 and the rigidity of the coil body 20. This reduces a difference in rigidity between the distal end part 12, where the coil body 20 is not placed, and the proximal end part 14, where the coil body 20 is placed, and consequently allows, for example, ensuring torquability of the catheter 100, as well as preventing occurrence of a kink, where the boundary site between the vicinity of the distal end and the vicinity of the proximal end of the catheter 100 is bent and no longer reverts to its original position due to the difference in rigidity.

Meanwhile, the scene of surgery requires to shorten procedure time. For this request, through a great deal of earnest investigation, the inventor found that proposal of a catheter accessible to a lesion without use of any guide wire allows procedure time to be more saved. In other words, the catheter 100 according to the embodiment has a distal end accessible to a lesion without use of any guide wire, has high performance of the distal end part 12 to select a smaller-diameter blood vessel, and high torquability from the proximal end part 14 to the distal end part 12, and is unlikely to generate a kink. Therefore, the catheter can be used for, e.g., catheter embolization such as locomotorium catheter therapy (TAME: Transcatheter arterial micro embolization), without use of any guide wire. For example, prolonged-pain diseases such as a frozen shoulder have an abnormal blood vessel growing and remaining, and the abnormal blood vessel causes the pain. The catheter 100 enables access to a lesion where an abnormal blood vessel remains, without use of any guide wire. Then, an embolic substance is injected into the abnormal blood vessel via a lumen S of the catheter 100, thereby allowing an abnormal blood vessel to diminish to improve pain.

In the embodiment, the extracoil braided portion 30A of the braided body 30 has the reduced diameter part 31C having an outer diameter reduced toward the distal end of the catheter 100, and the first constant diameter part 31A adjacent to the distal end of the reduced diameter part 31C and having the same outer diameter throughout the entire length in the axial direction of the catheter 100 (the same as the outer diameter of the distal end of the reduced diameter part 31C). With this, the embodiment can prevent the extracoil braided portion 30A from being easily pulled out from a blood vessel after insertion into the blood vessel, compared to a configuration including an extracoil braided portion 30A having an outer diameter reduced toward the distal end throughout the entire length of the extracoil braided portion 30A.

In the embodiment, in the catheter body 10, the inner diameter of the inner resin layer 40 in at least a part of the extracoil braided portion 30A (the first inner diameter E1 of the first inner peripheral surface 13A in the vicinity of the distal end) is smaller than the inner diameter of the inner resin layer 40 in the intracoil braided portion 30B (the second inner diameter E2 of the second inner peripheral surface 13B in the vicinity of the proximal end). With this, the embodiment includes a part with a relatively large inner diameter in the proximal end part 14 of the catheter 100, thus allows ensuring a large volume of accommodation space inside the catheter 100, and accommodating a large amount of fluid (liquid or gas, e.g., contrast medium) in the proximal end part 14 of the catheter 100, for example, when the fluid is injected from the distal end of the catheter 100. In addition, since the distal end part 12 of the catheter 100 includes a part with a relatively small inner diameter, the flow velocity of the fluid delivered from the proximal end part 14 of the catheter 100 can be increased at the distal end part 12 to improve the jet force of the fluid from the distal end of the catheter 100.

In the embodiment, the thickness of the vicinity of the distal end of the cushioning material 60 is thinner than the thickness of the vicinity of the proximal end of the cushioning material 60. With this, the embodiment further reduces a difference in rigidity between the distal end part 12 and the proximal end part 14 of the catheter 100, thereby allowing more effective prevention of occurrence of a kink, compared to a configuration having the cushioning material 60 with a uniform thickness.

In the embodiment, the cushioning material 60 has an extended portion 66 that extends so as to lie over a plurality of wires 22 that composes the coil body 20 as well as are adjacent to each other. Accordingly, in the embodiment, the cushioning material 60 has the extended portion 66, which causes a high adhesion between the cushioning material 60 and the coil body 20, thus further reducing a difference in rigidity between the distal end part 12 and the proximal end part 14 of the catheter 100, thereby allowing more effective prevention of occurrence of a kink.

### B. Second Embodiment:

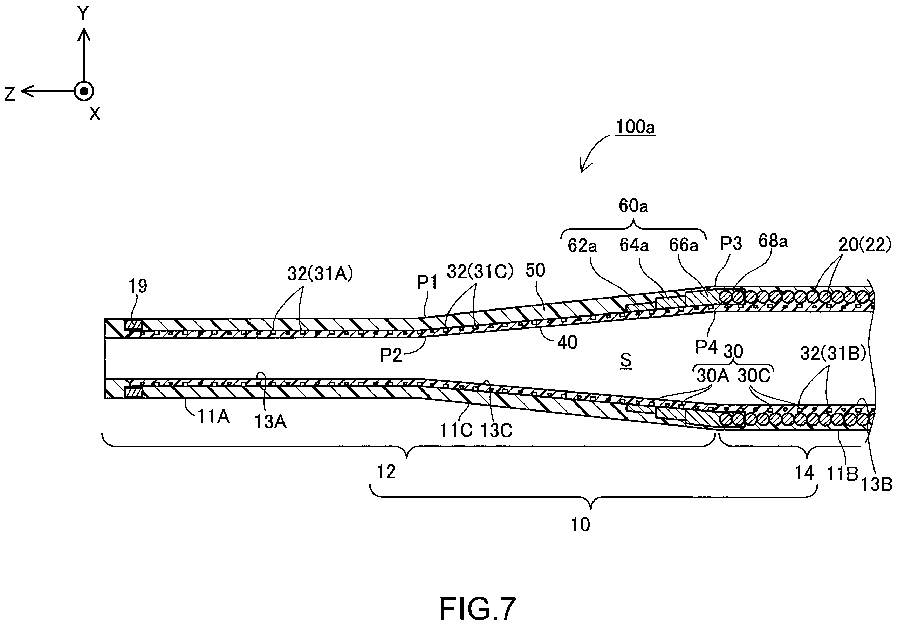

Fig. 7 is an explanatory diagram illustrating a longitudinal section configuration of a part of a catheter 100a in the second embodiment. In a configuration of the catheter 100a in the second embodiment, description will be omitted for the same configuration as in the catheter 100 in the first embodiment described above, by providing the same symbols.

The second embodiment is different from the first embodiment described above in that the boundary position P3 between the proximal end-side constant diameter part 11B and the connection part 11C, and the boundary position P4 between the second inner peripheral surface 13B and the third inner peripheral surface 13C are approximately the same in the axial direction of the catheter 100 (Z-axis direction). In addition, the distal end of the coil body 20 extends up to the boundary position P4 between the second inner peripheral surface 13B and the third inner peripheral surface 13C, thus allowing effective improvement of torquability from the proximal end part 14 to the distal end part 12 of the catheter 100, compared to a configuration where the distal end of the coil body 20 does not extend up to the position P4.

In the embodiment, the cushioning material 60a is placed so as to surround the outer periphery of the reduced diameter part 31C in the extracoil braided portion 30A and to cause the inner peripheral surface of the cushioning material 60 to be in contact with the outer peripheral surface of the reduced diameter part 31C. Such configuration allows preventing the cushioning material 60a from separating from the coil body 20 and the braided body 30. That is, for example, in a process of insertion of the catheter body 10 into the body, the cushioning material 60a receives push pressure from the outer resin layer 50, thereby causing the cushioning material 60 to be pressed against the distal end of the coil body 20 and the braided body 30, and thus allowing prevention of separation of the cushioning material 60a from the coil body 20 and the braided body 30.

The thickness of the vicinity of the distal end of the cushioning material 60a is thinner than the thickness of the vicinity of the proximal end of the cushioning material 60a. Particularly, the cushioning material 60a has a first cushioning material 62a, a second cushioning material 64a, and a third cushioning material 66a. The second cushioning material 64a is located in the vicinity of the proximal end of the first cushioning material 62a, and the thickness of the second cushioning material 64a is thicker than the thickness of the first cushioning material 62a. The third cushioning material 66a is located in the vicinity of the proximal end of the second cushioning material 64a, and the thickness of the third cushioning material 66a is thicker than the thickness of the second cushioning material 64a. With this, the embodiment further reduces a difference in rigidity between the distal end part 12 and the proximal end part 14 of the catheter 100a, and allows more effective prevention of occurrence of a kink, compared to a configuration having the cushioning material 60a with a uniform thickness. The first cushioning material 62a, the second cushioning material 64a, and the third cushioning material 66a are preferably in contact with each other, and they may also be individually present or integrally bonded.

The cushioning material 60a has an extended portion 68a that extends so as to lie over a plurality of wires 22 that composes the coil body 20 as well as are adjacent to each other. This results in higher adhesion between the cushioning material 60a and the coil body 20. The extended portion 68a is also formed over the entire circumference of the coil body 20.

### C. Third Embodiment:

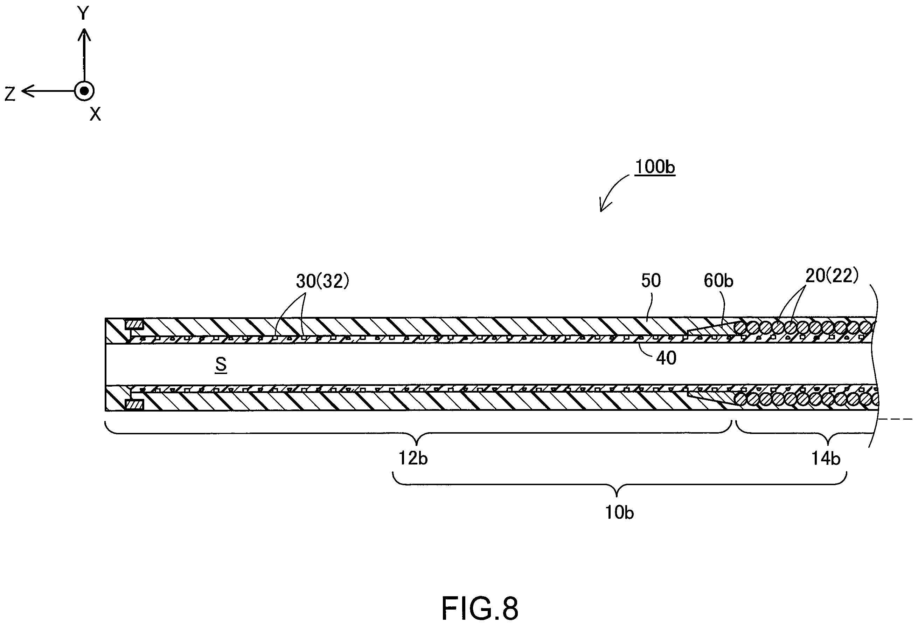

Fig. 8 is an explanatory diagram illustrating a longitudinal section configuration of a part of a catheter 100b in the third embodiment. In a configuration of the catheter 100b in the third embodiment, description will be omitted for the same configuration as in the catheter 100 in the first embodiment described above, by providing the same symbols.

The third embodiment is different from the first embodiment described above in that the catheter body 10b of the catheter 100b has approximately the same inner diameter and the same outer diameter throughout the entire length in the axial direction (Z-axial direction). The cushioning material 60b also has a thickness becoming continuously thinner toward the distal end. With this, the embodiment further reduces a difference in rigidity between the distal end part 12b and the proximal end part 14b of the catheter 100b, thereby allowing more effective prevention of occurrence of a kink, compared to a configuration having a thickness becoming thinner in stages.

### D. Fourth Embodiment:

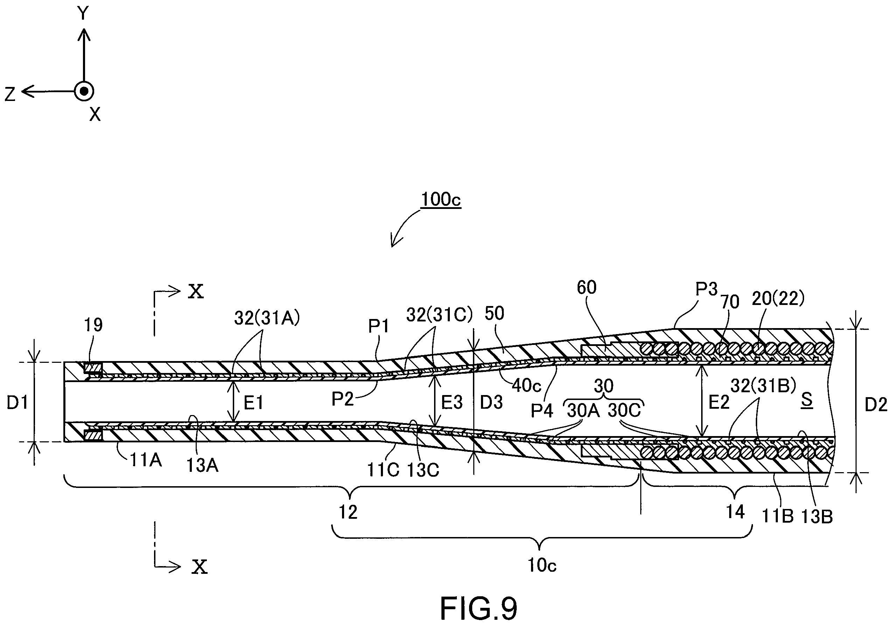

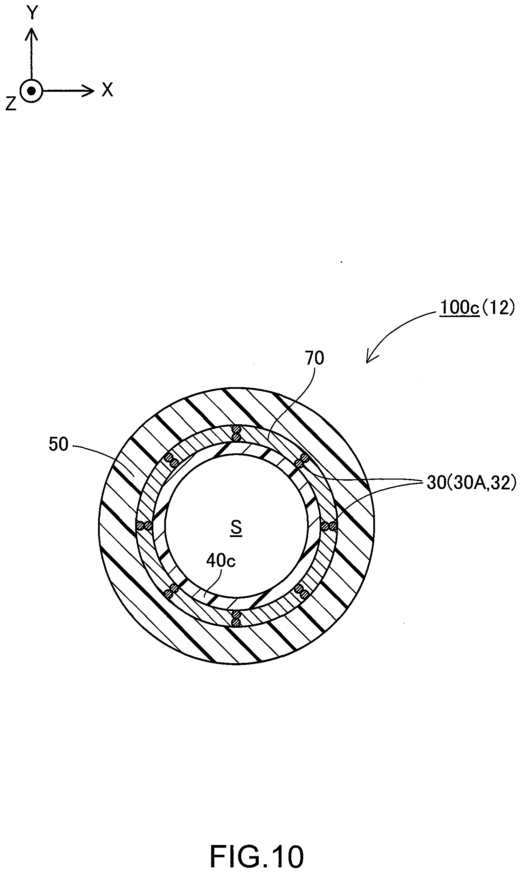

Fig. 9 is an explanatory diagram illustrating a longitudinal section configuration of a part of a catheter 100c in the fourth embodiment, and Fig. 10 is a transverse sectional view at the position X-X. In a configuration of the catheter 100c in the fourth embodiment, description will be omitted for the same configuration as in the catheter 100 in the first embodiment described above, by providing the same symbols.

The fourth embodiment is different from the first embodiment described above in that the catheter body 10c of the catheter 100c includes a medium resin layer 70, which is formed by filing a resin in gaps in the braided body 30, throughout the entire length in the axial direction (Z-axial direction). That is, in the fourth embodiment, the inner resin layer 40c is formed so as to coat the inner peripheral surface of the braided body 30, but does not enter the gaps of the braided body 30. Instead, the medium resin layer 70 is formed by filling the gaps of the braided body 30. The medium resin layer 70 is in contact with both the inner resin layer 40c and the outer resin layer 50. When different resins are employed for the inner resin layer 40c and the outer resin layer 50 and do not have high adhesiveness, employment of a resin having high adhesiveness for both of the inner resin layer 40c and the outer resin layer 50 as a resin of the medium resin layer 70 allows the medium resin layer 70 to improve adhesiveness between the resin layers.

### E. Fifth Embodiment:

### E-1. Overall Configuration of Catheter 200:

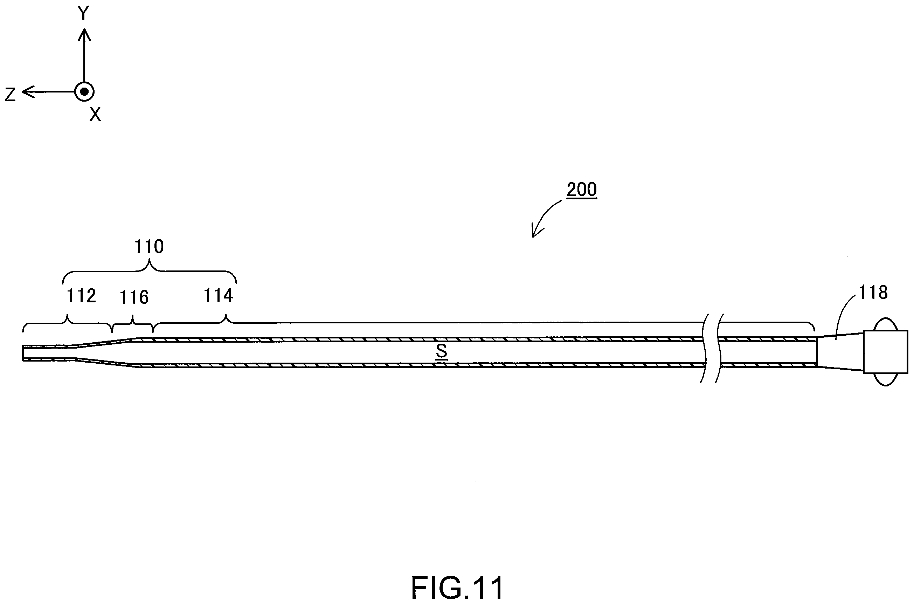

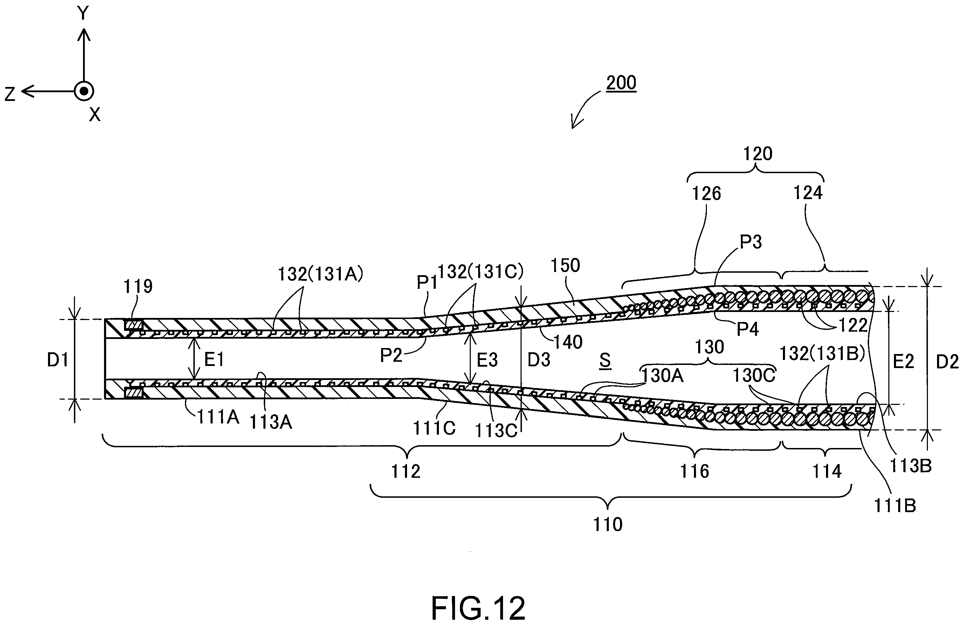

Fig. 11 is an explanatory diagram schematically illustrating a longitudinal section configuration of a catheter 200 in a fifth embodiment. Fig. 11 omits the detailed configuration of a catheter body 110, but Fig. 12 shows a longitudinal section of a part of the catheter body 110 in an enlarged view. Although Fig. 11 and Fig. 12 show the catheter 200 in an entirely straight form parallel to the Z-axis direction, the catheter 200 has enough flexibility to be bent.

The catheter 200 includes the catheter body 110, and a connector 118, which is attached to the proximal end thereof. The catheter body 110 has a catheter distal end part 112, a catheter proximal end part 114, and a catheter connection part 116.

The catheter distal end part 112 is a part that includes the distal end of the catheter body 110 and has relatively high flexibility due to absence of a coil body 120. The catheter proximal end part 114 is a part that includes the proximal end of the catheter body 110 and has relatively high rigidity due to presence of a proximal end-side coil 124 in the coil body 120. The catheter connection part 116 is a part that connects the catheter distal end part 112 to the catheter proximal end part 114 in the axial direction of the catheter 200, and is a part for reducing a difference in rigidity between the catheter distal end part 112 and the catheter proximal end part 114 by including a connection-side coil 126 described later in the coil body 120.

As shown in Figs. 11 and 12, the catheter body 110 is a tubular (e.g., cylindrical) member with openings at the distal end and proximal end. Inside the catheter body 110, a lumen S is formed with extending from the distal end to the proximal end of the catheter body 110.

As shown in Fig. 12, the catheter body 110 has a distal end-side constant diameter part 111A, a proximal end-side constant diameter part 111B, and a connection part 111C. The distal end-side constant diameter part 111Ais a part that includes the distal end of the catheter body 110, and the first outer diameter D1 of the distal end-side constant diameter part 111A is approximately the same throughout the entire length in the axial direction of the catheter 200. The proximal end-side constant diameter part 111B is a part that includes the proximal end of the catheter body 110, and the second outer diameter D2 of the proximal end-side constant diameter part 111B is larger than the first outer diameter D1 of the distal end-side constant diameter part 111A and approximately the same throughout the entire length in the axial direction of the catheter 200. The connection part 111C is a part that is located between the distal end-side constant diameter part 111A and the proximal end-side constant diameter part 111B, and connects the distal end-side constant diameter part 111A to the proximal end-side constant diameter part 111B, and the third outer diameter D3 of the connection part 111C continuously increases from the distal end side toward the proximal end side. In the embodiment, the distal end-side constant diameter part 111A composes the catheter distal end part 112, the proximal end-side constant diameter part 111B composes the catheter proximal end part 114, and the connection part 111C composes the catheter connection part 116.

The first inner diameter E1 of a first inner peripheral surface 113Ain the vicinity of the distal end of the catheter body 110 is approximately the same throughout the entire length in the axial direction of the catheter 200 (Z-axis direction) in the first inner peripheral surface 113A. The second inner diameter E2 of a second inner peripheral surface 113B in the vicinity of the proximal end of the catheter body 110 is larger than the first inner diameter E1 of the first inner peripheral surface 113A, and approximately the same throughout the entire length in the axial direction of the catheter 200 in the second inner peripheral surface 113B. A third inner peripheral surface 113C, which is located between the first inner peripheral surface 113A and the second inner peripheral surface 113B, increase continuously from the distal end side toward the proximal end side.

In the embodiment, the boundary position P1 between the distal end-side constant diameter part 111A and the connection part 111C, and the boundary position P2 between the first inner peripheral surface 113A and the third inner peripheral surface 113C are approximately the same in the axial direction of the catheter 200 (Z-axis direction). The boundary position P3 between the proximal end-side constant diameter part 111B and the connection part 111C, and the boundary position P4 between the second inner peripheral surface 113B and the third inner peripheral surface 113C are approximately the same in the axial direction.

### E-2. Detailed Configuration of Catheter Body 110:

As shown in Fig. 12, the catheter body 110 includes the coil body 120, a braided body 130, an inner resin layer 140, and an outer resin layer 150. The catheter 200 is different from those in the first to fourth embodiments described above in that it includes no cushioning material.

The coil body 120 is a coiled member formed as a hollow cylindrical shape by spirally winding a plurality of wires 122. The coil body 120 is not placed in the catheter distal end part 112 in the catheter body 110, but is placed in the catheter proximal end part 114 and the catheter connection part 116.

The wire 122 may employ a metallic material. Examples of the metallic materials to be used can include radiopaque alloy such as stainless steel (SUS302, SUS304, SUS316, etc.), superelastic alloy such as Ni-Ti alloy, a piano wire, nickel-chromium-based alloy, or cobalt alloy; and radiopaque alloy such as gold, platinum, tungsten, or alloy containing these elements (e.g., platinum-nickel alloy). The outer diameter of the wire 122 is preferably larger than the outer diameter of a wire 132, which composes the braided body 130 described next. Furthermore, the rigidity of the wire 122 is preferably larger than the rigidity of the wire 132.

The braided body 130 is a tubular member derived by braiding a plurality of wires 132. The braided body 130 has a mesh-like structure in which a plurality of wires 132 is braided so as to intersect each other (see e.g., Fig. 3). As shown in Fig. 12, the braided body 130 is continuously placed from the proximal end to the distal end in the catheter body 110. The braided body 130 has approximately the same thickness throughout the entire length in the axial direction of the catheter 200.

The braided body 130 has an extracoil braided portion 130A and an intracoil braided portion 130B. The intracoil braided portion 130B is placed on the inner peripheral side of the coil body 120. The extracoil braided portion 130A is placed so as to extend from the intracoil braided portion 130B toward the distal end beyond the coil body 120. In other words, the coil body 120 is not placed on the outer periphery of the extracoil braided portion 130A in the braided body 130, but is placed so as to surround the outer periphery of the intracoil braided portion 130B.

The braided body 130 is shaped along the inner peripheral surface of the catheter body 110 (the first inner peripheral surface 113A, the second inner peripheral surface 113B, the third inner peripheral surface 113C). Particularly, in the braided body 130, a part located on the outer periphery of the first inner peripheral surface 113A corresponds to a first constant diameter part 131A, which has approximately the same outer diameter throughout the entire length in the axial direction of the catheter 200. In the braided body 130, a part located on the outer periphery of the second inner peripheral surface 113B corresponds to a second constant diameter part 131B, which has an outer diameter that is larger than the outer diameter of the first constant diameter part 131A and approximately the same throughout the entire length in the axial direction of the catheter 200. In the braided body 130, a part located on the outer periphery of the third inner peripheral surface 113C corresponds to a reduced diameter part 131C, which has an outer diameter continuously reduced toward the distal end. In the embodiment, the first constant diameter part 131A and the vicinity of the distal end of the reduced diameter part 131C compose the extracoil braided portion 130A as well as are included in the catheter distal end part 112 and the distal end side of the catheter connection part 116, and the vicinity of the proximal end of the reduced diameter part 131C and the second constant diameter part 131B compose the intracoil braided portion 30B as well as are included in the vicinity of the proximal end of the catheter connection part 116 and the catheter proximal end part 114.

The wire 132 can employ a metallic material. Examples of the metallic materials to be used can include tungsten and stainless steel (SUS 302, SUS 304, SUS 316, etc.)

The inner resin layer 140 coats the inner peripheral surface of the braided body 130. Particularly, the inner resin layer 140 is continuously placed from the proximal end to the distal end of the catheter body 110, and coats the inner peripheral surface side of the braided body 130 throughout the entire length. In other words, the inner resin layer 140 composes the inner peripheral surface of the catheter body 110 (the inner peripheral surfaces 113A, 113B, and 113C as described above). The thickness of the inner resin layer 140 is approximately the same throughout the entire length in the axial direction of the catheter 200.

In the catheter body 110, the inner diameter of the inner resin layer 140 in at least a part of the extracoil braided portion 130A (the first inner diameter E1 of the first inner peripheral surface 113A in the vicinity of the distal end) is smaller than the inner diameter of the inner resin layer 140 in the intracoil braided portion 130B (the second inner diameter E2 of the second inner peripheral surface 113B in the vicinity of the proximal end). The inner resin layer 140 is also in close contact with the inner peripheral surface of the braided body 130, as well as enter each gap between the wires 132 composing the braided body 130. The inner resin layer 140 is composed of e.g., polytetrafluoroethylene (PTFE) resin.

The outer resin layer 150 coats the outer peripheral surface of the extracoil braided portion 130A of the braided body 130, and the coil body 120. Particularly, the outer resin layer 150 is continuously placed from the proximal end to the distal end of the catheter body 110, and coats the outer peripheral surface side of the extracoil braided portion 130A and the coil body 120 throughout the entire length. In other words, the outer resin layer 150 composes the outer peripheral surface of the catheter body 110. The outer resin layer 150 has an approximately the same thickness throughout the entire length in the axial direction of the catheter 200. The outer resin layer 150 is composed of e.g., polytetrafluoroethylene (PTFE) resin. The inner resin layer 140 and the outer resin layer 150 may be composed of the same type of resin as each other, or different types of resin from each other. The distal end part in the outer resin layer 150 has an annular chip 119 embedded therein.

The coil body 120 has a proximal end-side coil 124 and a connection-side coil 126. The outer diameter of the wire 122 composing the proximal end-side coil 124 is approximately the same throughout the entire length in the axial direction of the catheter 200. In other words, the flexural rigidity of the proximal end-side coil 124 is approximately the same throughout the entire length in the axial direction. The wire 122 composing the connection-side coil 126 becomes continuously thinner toward the distal end of the connection-side coil 126 (coil body 120). In other words, the flexural rigidity of the connection-side coil 126 becomes lower toward the distal end of the connection-side coil 126. The wire 122 may become thinner in stages toward the distal end of the connection-side coil 126. The distal end of the proximal end-side coil 124 and the proximal end of the connection-side coil 126 are continuously and integrally connected.

The position of the proximal end of the catheter distal end part 112 is located closer to the distal end than the position P3. The proximal end-side coil 124 is placed in the catheter proximal end part 114. The connection-side coil 126 is placed in the catheter connection part 116.

Relation of flexural rigidity among the catheter distal end part 112, the catheter proximal end part 114, and the catheter connection part 116 (hereinafter referred to as "defined relation of flexural rigidity") is as follows:
"the flexural rigidity of the catheter distal end part": 0.005 gf·cm²/cm or more to less than 0.05 gf·cm²/cm;
"the flexural rigidity of the catheter connection part": 0.05 gf·cm²/cm or more to less than 1.4 gf·cm²/cm; and
"the flexural rigidity of the catheter proximal end part": 1.4 gf·cm²/cm or more to 3.0 gf·cm²/cm or less.

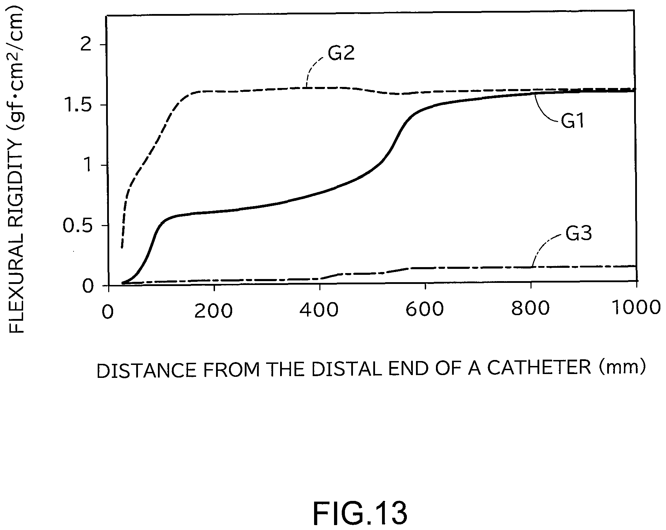

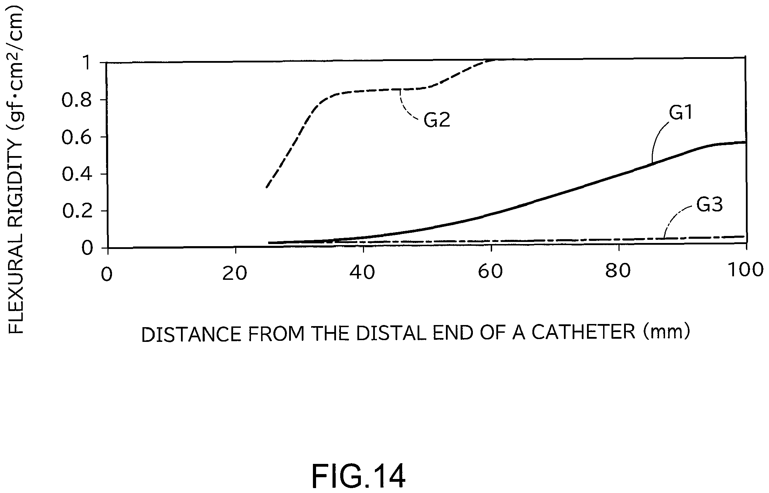

Fig. 13 is a graph showing the overall flexural rigidity of the catheter 200, and Fig. 14 is a graph showing flexural rigidity of a catheter distal end part 112 and a catheter connection part 116 in a catheter 200. The ordinate axis of each figure represents flexural rigidity of a catheter (gf·cm²/cm), and the abscissa axis represents distance from the distal end of a catheter (mm). Graph G1 in each figure indicates flexural rigidity of the catheter 200, graph G2 indicates flexural rigidity of a known guiding catheter, and graph G3 indicates flexural rigidity of a known microcatheter.

The flexural rigidity of the catheter 200 can be derived by the following method. In detail, while a part of the proximal end side of a measured site in the catheter 200 is fixed unmovably via a torque gage, a part of the distal end side of the measured site in the catheter 200 is fixed to a movable chuck, under ordinary temperature. Then, the movable chuck is moved so as to draw a curvature track in each of two directions perpendicular to the axis of the catheter 200. In this manner, the catheter 200 was oscillated by 90 degrees to each of the two directions to induce bending deformation. Measurement is made for the maximum value of torque load measured with the torque gage at that time, and flexural rigidity is derived from the measuring results. Flexural rigidity of the guiding catheter or the microcatheter can also be derived by a similar method thereof.

As can be seen in graph G1 and graph G2 in Fig. 13, the catheter proximal end part 114 of the catheter 200 has approximately equivalent rigidity to that of the guiding catheter. Graph G1 and graph G3 in Fig. 13 and Fig. 14 indicates that the catheter distal end part 112 of the catheter 200 has approximately equivalent rigidity to those of the distal end part of the microcatheter. As can also be seen, in the catheter 200, the presence of the catheter connection part 116 continuously increase flexural rigidity from the proximal end of the catheter distal end part 112 toward the distal end of the catheter proximal end part 114.

### E-3. Effect of the Embodiment:

As shown in Fig. 12, in the catheter 200 according to the embodiment, the coil body 120 is not placed in the vicinity of the distal end of the catheter 200 (the catheter distal end part 112 where the extracoil braided portion 130A is located), and the coil body 120 (proximal end-side coil 124) is placed in the vicinity of the proximal end of the catheter 200 (the catheter proximal end part 114 where the intracoil braided portion 130B is located). Thus, the vicinity of the catheter distal end part 112 has relatively high flexibility, and the catheter proximal end part 114 has relatively high rigidity. This allows providing improved performance of the catheter distal end part 112 to select a smaller-diameter blood vessel, and improved torquability from the catheter proximal end part 114 to the catheter distal end part 112.

The catheter 200 has a catheter connection part 116 that connects the catheter distal end part 112 and the catheter proximal end part 114. The catheter connection part 116 has rigidity between the rigidity of the catheter distal end part 112 and the rigidity of the catheter proximal end part 114 (the defined relation of flexural rigidity described above). This reduces a difference in rigidity between the catheter distal end part 112, where the coil body 120 is not placed, and the catheter proximal end part 114, where the coil body 120 is placed, and consequently allows, for example, ensuring torquability of the catheter 200, as well as preventing occurrence of a kink, where the boundary site between the vicinity of the distal end and the vicinity of the proximal end of the catheter 200 is bent and no longer reverts to its original position due to the difference in rigidity.

Generally, in a technique of advancing a catheter and a guide wire into a blood vessel, a guiding catheter is inserted into the blood vessel, and then a microcatheter and a guide wire are inserted into the guiding catheter and advanced alternately. By contrast, when the catheter 220 is used, use of the catheter 200 and a guide wire allows execution of operation to alternately advance the guide wire and the catheter 200 without need for any guiding catheter. In particular, this eliminates need of a guiding catheter, when the catheter 200 advances through a relatively straight blood vessel from a puncture site to a treated site, i.e., with selecting a branched blood vessel once or twice, particularly, for catheter embolization such as locomotorium catheter therapy (TAME).

That is, in the catheter 200 of the embodiment, the catheter distal end part 112 has flexural rigidity as low as the level of the microcatheter, and the catheter proximal end part 114 has flexural rigidity as high as the level of the guiding catheter. The catheter connection part 116 connecting them offsets a difference in rigidity between the catheter proximal end part 114 and the catheter distal end part 112, thus preventing occurrence of a kink of the catheter 200 due to the difference in rigidity between the catheter distal end part 112 and the catheter proximal end part 114, as well as allowing execution of operation of alternately advancing a guide wire and the catheter 200 without use of any guiding catheter with a specific technique.

### F. Sixth Embodiment:

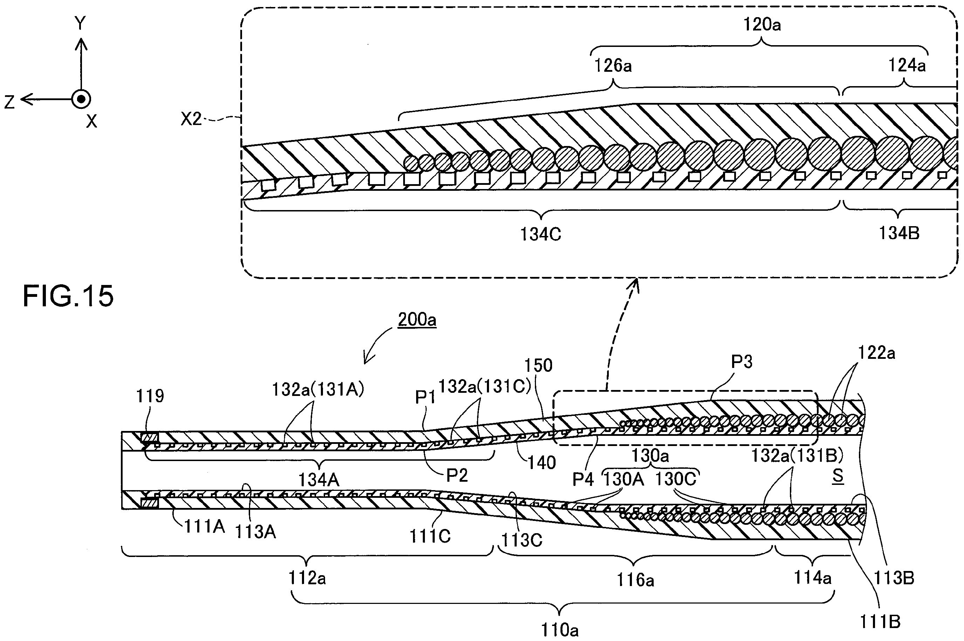

Fig. 15 is an explanatory diagram illustrating a longitudinal section configuration of a part of a catheter 200a in the sixth embodiment. This figure further shows a configuration of a part X2 in the catheter 200a (a connection-side coil 126a and a connection-side braided body 134C described later) in an enlarged view. In a configuration of the catheter 200a in the sixth embodiment, description will be omitted for the same configuration as in the catheter 200 in the fifth embodiment described above, by providing the same symbols.

The sixth embodiment is different from the fifth embodiment described above in that the boundary position P3 between the proximal end-side constant diameter part 111B and the connection part 111C is located closer to the proximal end than the boundary position P4 between the second inner peripheral surface 113B and the third inner peripheral surface 113C. The distal end of a coil body 120a is located closer to the distal end of the catheter 200a than the position P3 in the axial direction of the catheter 200a (Z-axis direction). This more effectively improves torquability from the catheter proximal end part 114a to the catheter distal end part 112a of the catheter 200. The distal end of the coil body 120a is also located closer to the proximal end than the position P4. This prevents increase in the outer diameters of the distal end-side constant diameter part 111A, the connection part 111C, and the like due to presence of the coil body 120a.

The catheter 200a has a catheter distal end part 112a, a catheter proximal end part 114a, and a catheter connection part 116a.

The catheter distal end part 112a is a part that includes the distal end of the catheter body 110a and has relatively high flexibility due to absence of a coil body 120a, as is in the catheter distal end part 112 of the fifth embodiment described above. The catheter proximal end part 114a is a part that includes the proximal end of the catheter body 110a and has relatively high rigidity due to presence of a proximal end-side coil 124a in the coil body 120a, as is in the catheter proximal end part 114 of the fifth embodiment described above. The catheter connection part 116a is a part that connects the catheter distal end part 112a to the catheter proximal end part 114a in the axial direction of the catheter 200a, and is a part for reducing a difference in rigidity between the catheter distal end part 112a and the catheter proximal end part 114a by including the connection-side coil 126a and a connection-side braided body 134C, as descried later. Specific description will be provided below.

The coil body 120a has a proximal end-side coil 124a and a connection-side coil 126a. The proximal end-side coil 124a has approximately the same configuration as the coil body 120 in the fifth embodiment described above. The diameter of the wire 122a composing the proximal end-side coil 124a is approximately the same throughout the entire length in the axial direction of the catheter 200a. In other words, the flexural rigidity of the proximal end-side coil 124a is approximately the same throughout the entire length in the axial direction. The wire 122a composing the connection-side coil 126a becomes continuously thinner toward the distal end of the connection-side coil 126a (coil body 120a). In other words, the flexural rigidity of the connection-side coil 126a becomes lower toward the distal end of the connection-side coil 126a. The wire 122a may become thinner in stages toward the distal end of the connection-side coil 126a. The distal end of the proximal end-side coil 124a and the proximal end of the connection-side coil 126a are continuously and integrally connected.

The braided body 130a has a distal end-side braided body 134A, a rear end-side braided body 134B, and a connection-side braided body 134C. The distal end-side braided body 134A is a member that includes the distal end of the braided body 130a as well as is located closer to the distal end than the coil body 120a, and the diameter of the wire 132a composing the distal end-side braided body 134A is approximately the same throughout the entire length in the axial direction of the distal end-side braided body 134A. In other words, the flexural rigidity of the distal end-side braided body 134A is approximately the same throughout the entire length in the axial direction. The rear end-side braided body 134B is a member that includes the rear end of the braided body 130a as well as is located on the inner peripheral side of the coil body 120a, and the diameter of the wire 132a composing the rear end-side braided body 134B is approximately the same throughout the entire length in the axial direction of the rear end-side braided body 134B. In other words, the flexural rigidity of the rear end-side braided body 134B is approximately the same throughout the entire length in the axial direction. Note that the diameter of the wire 132a composing the distal end-side braided body 134A and the diameter of the wire 132a composing the rear end-side braided body 134B are approximately the same.

The connection-side braided body 134C is a part that connects the distal end-side braided body 134A to the rear end-side braided body 134B in the axial direction. The distal end side of the connection-side braided body 134C is protruded from the coil body 120a toward the distal end, and the vicinity of the proximal end of the connection-side braided body 134C is placed on the inner peripheral side of the coil body 120a. The wire 132a composing the connection-side braided body 134C becomes thicker as closer to the distal end of the coil body 120a from the distal end of the connection-side braided body 134C. In other words, the flexural rigidity of the connection-side braided body 134C becomes continuously higher as closer to the distal end of the coil body 120a from the distal end of the connection-side braided body 134C. Moreover, the wire 132a composing the connection-side braided body 134C becomes thicker as closer to the distal end of the coil body 120a from the proximal end of the connection-side braided body 134C. In other words, the flexural rigidity of the connection-side braided body 134C becomes continuously higher as closer to the distal end of the coil body 120a from the proximal end of the connection-side braided body 134C. Note that the flexural rigidity of the connection-side braided body 134C may become higher in stages from the distal or proximal end of the connection-side braided body 134C to the distal end of the coil body 120a. The proximal end of the distal end-side braided body 134A and the distal end of the connection-side braided body 134C are continuously and integrally connected, and the proximal end of the connection-side braided body 134C and the distal end of the rear end-side braided body 134B are continuously and integrally connected.

The distal end-side braided body 134A is placed in the catheter distal end part 112a. The proximal end-side coil 124a and the rear end-side braided body 134B are in the catheter proximal end part 114a. The connection-side coil 126a and the connection-side braided body 134C are placed in the catheter connection part 116a.

The flexural rigidities in the catheter distal end part 112a, the catheter proximal end part 114a, and the catheter connection part 116a are the same as the defined relation of flexural rigidity in the fifth embodiment described above.

As shown in Fig. 15, in the catheter 200a, a part of the coil body 120a (connection-side coil 126a) is placed in the catheter connection part 116a. The wire 122a composing the connection-side coil 126a becomes thinner toward the distal end of the catheter connection part 116a. Moreover, a part of the extracoil braided portion 130A (connection-side braided body 134C) is placed in the catheter connection part 116a, and the diameter of the wire 132a composing the vicinity of the distal end of the connection-side braided body 134C increases from the distal end of the connection-side braided body 134C toward the distal end of the coil body 120a. This allows effective reduction of a difference in rigidity between the catheter proximal end part 114a and the catheter connection part 116a.

In the embodiment, the diameter of the wire 132a composing the vicinity of the proximal end of the connection-side braided body 134C increases from the proximal end of the connection-side braided body 134C toward the distal end of the coil body 120a. Thus, in the catheter connection part 116a, the connection-side coil 126a including a relatively smaller diameter of the wire 122a composing the coil body 120a is reinforced with the connection-side braided body 134C.

### G. Seventh Embodiment:

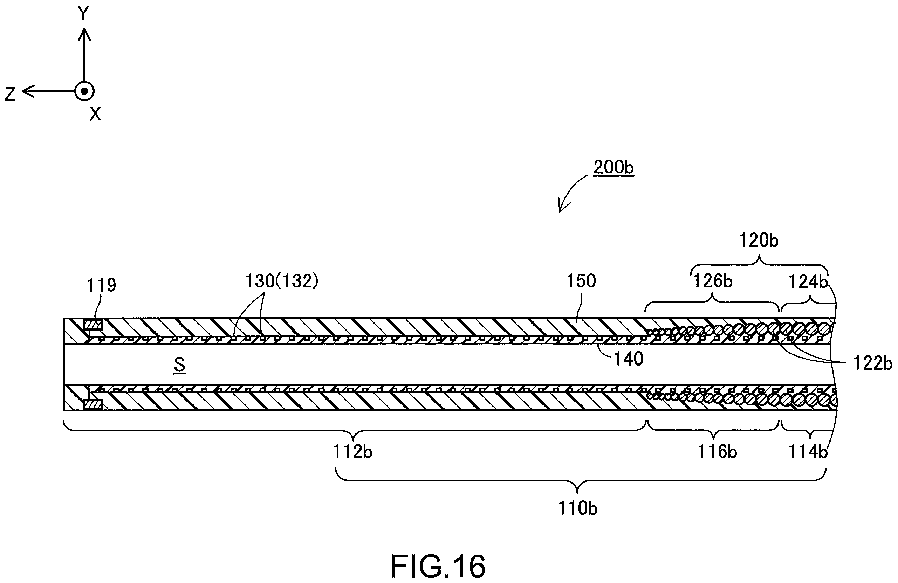

Fig. 16 is an explanatory diagram illustrating a longitudinal section configuration of a part of a catheter 200b in the seventh embodiment. In a configuration of the catheter 200b in the seventh embodiment, description will be omitted for the same configuration as in the catheter 200 in the fifth embodiment described above, by providing the same symbols.

The catheter body 110b of the catheter 200b is different from the fifth embodiment described above in that it has approximately the same inner diameter and the same outer diameter throughout the entire length in the axial direction (Z-axial direction). The catheter 200b has a catheter distal end part 112b, a catheter proximal end part 114b, and a catheter connection part 116b.

The catheter distal end part 112b is a part that includes the distal end of the catheter body 110b and has relatively high flexibility due to absence of a coil body 120b described later. The catheter proximal end part 114b is a part that includes the proximal end of the catheter body 110b and is a part that has relatively high rigidity due to presence of a proximal end-side coil 124b described later, in the coil body 120b. The catheter connection part 116b is a part that connects the catheter distal end part 112b to the catheter proximal end part 114b in the axial direction of the catheter 200b, and is a part for reducing a difference in rigidity between the catheter distal end part 112b and the catheter proximal end part 114b by including a connection-side coil 126b descried later in the coil body 120b. Specific description will be provided below.

The coil body 120b has a proximal end-side coil 124b and a connection-side coil 126b. The proximal end-side coil 124b has approximately the same configuration as the coil body 20b in the third embodiment described above. The diameter of the wire 122b composing the proximal end-side coil 124b is approximately the same throughout the entire length in the axial direction of the catheter 200b. In other words, the flexural rigidity of the proximal end-side coil 124b is approximately the same throughout the entire length in the axial direction. The wire 122b composing the connection-side coil 126b becomes continuously thinner toward the distal end of the connection-side coil 126b (coil body 120b). In other words, the flexural rigidity of the connection-side coil 126b becomes lower toward the distal end of the connection-side coil 126b. The wire 122b may become thinner in stages toward the distal end of the connection-side coil 126b. The distal end of the proximal end-side coil 124b and the proximal end of the connection-side coil 126b are continuously and integrally connected.

The proximal end-side coil 124b is placed in the catheter proximal end part 114b. The connection-side coil 126b is placed in the catheter connection part 116b.

The relation of flexural rigidities in the catheter distal end part 112b, the catheter proximal end part 114b, and the catheter connection part 116b are the same as the defined relation of flexural rigidity in the fifth embodiment described above.

In the configuration including the same outer diameter of the catheter 200b throughout the entire length, the catheter 200b according to the embodiment allows ensuring torquability of the catheter 200b, as well as preventing occurrence of a kink of the catheter 200b due to a difference in rigidity between the catheter distal end part 112b and the catheter proximal end part 114b.

### H. Modified Examples:

The technology disclosed herein is not limited to the embodiments described above, and can be modified to a variety of aspects within the range not departing from its spirits; for example, the following modification is also available.

In the embodiment described above, the coil body 20, 120, 120a, or 120b is a tubular member wound with a plurality of wires 22, 122, 122a, or 122b, but the coil body 20, 120, 120a, or 120b may also be a coiled member formed into a hollow cylindrical shape by spirally winding a single wire 22, 122, 122a, or 122b. Furthermore, a tubular member produced by spirally winding one or more twisted wires derived by twisting together a single wire 22, 122, 122a, or 122b or a plurality of wires 22, 122, 122a, or 122b may be used for the coil body 20, 120, 120a, or 120b.

The configuration of the catheter 100, 100a, 100b, 100c, 200, 200a, or 200b in the embodiments is just an example, and can be modified variously. For example, in the embodiment described above, all of the coil body 20, 120, 120a, or 120b, the braided body 30, 130, or 130a, the inner resin layer 40 or 140, the outer resin layer 50 or 150, the cushioning material 60, 60a, or 60b, and the medium resin layer 70 have been formed as a single piece, but is not limited thereto, and may be composed of a plurality of pieces.

The fourth embodiment described above includes the medium resin layer 70, and the first to third embodiments have a resin filled in gaps in the braided body 30 to form the inner resin layer 40. These forms allow preventing the resin filled in the gaps in braided body 30 from disrupting the braided body 30. There is no limitation to these forms, and a resin forming the outer resin layer 50 may be filled in the gaps in the braided body 30.

The surface of the inner resin layer 40 and the outer resin layer 50 in the first to fourth embodiments described above and the medium resin layer 70 in the fourth embodiment may be subjected to surface finishing so as to improve adhesiveness to other layers.

In the first and sixth embodiments, for example, the boundary position P1 between the distal end-side constant diameter part 11A or 111A and the connection part 11C or 111C may be located closer to the distal end or the proximal end of the catheter 100 or 200a than the boundary position P2 between the first inner peripheral surface 13A or 113A and the third inner peripheral surface 13C or 113C. The boundary position P3 between the proximal end-side constant diameter part 11B or 111B and the connection part 11C or 111C may also be located in the same position as, or closer to the distal end than, the boundary position P4 between the second inner peripheral surface 13B or 113B and the third inner peripheral surface 13C or 113C.

In the first to fourth embodiments described above, the shape of the cushioning materials 60, 60a, and 60b have been annular in the axial view, but are not limited thereto, and may have a configuration that partially coats the outer periphery of the braided body 30. The cushioning material 60, 60a, and 60b may also have approximately the same thickness throughout the entire length.

In the first, second, and fourth embodiments described above, the reduced diameter part 31C in the braided body 30 may have a configuration with an outer diameter increasing in stages from the distal end side toward the proximal end side.

In the sixth embodiment described above, the configuration may be such that the connection-side coil 126a is not included, and that the wire 122a composing the coil body 120a is approximately the same throughout the entire length in the axial direction.

In the fifth to seventh embodiments described above, the configurations meet the defined relation of flexural rigidity described above by including the connection-side coil 126, 126a, or 126b, the connection-side braided body 134C, or the like, but the defined relation of flexural rigidity described above may also be met by altering at least one of Young's modulus of a resin material forming the inner resin layer 140 and the outer resin layer 150, and cross-sectional secondary moment defined by the cross-sectional shape and size of a member (at least one of the cross-sectional shape and the cross-sectional size).

The material of each member in the catheter 100, 100a, 100b, 100c, 200, 200a, and 200b in the embodiments described above is just an example, and can be modified variously.

### DESCRIPTION OF REFERENCE NUMERALS

10: catheter body (first and second embodiments)
10b: catheter body (third embodiment)
10c: catheter body (fourth embodiment)
11A: distal end-side constant diameter part (first to fourth embodiments)
11B: proximal end-side constant diameter part (first to fourth embodiments)
11C: connection part (first to fourth embodiments)
12: distal end part (first and second embodiments)
12b: distal end part (third embodiment)
13A: first inner peripheral surface (first to fourth embodiments)
13B: second inner peripheral surface (first to fourth embodiments)
13C: third inner peripheral surface (first to fourth embodiments)
14: proximal end part (first and second embodiments)
14b: proximal end part (third embodiment)
18: connector (first to fourth embodiments)
19: chip (first to fourth embodiments)
20: coil body (first to fourth embodiments)
22: wire (coil body) (first to fourth embodiments)
30: braided body (first to fourth embodiments)
30A: extracoil braided portion (first to fourth embodiments)
30B: intracoil braided portion (first to fourth embodiments)
31A: first constant diameter part (first to fourth embodiments)
31B: second constant diameter part (first to fourth embodiments)
31C: reduced diameter part (first to fourth embodiments)
32: wire (braided body) (first to fourth embodiments)
40: inner resin layer (first to third embodiments)
40c: inner resin layer (fourth embodiment)
50: outer resin layer (first to fourth embodiment)
60: cushioning material (first and fourth embodiments)
60a: cushioning material (second embodiment)
60b: cushioning material (third embodiment)
62: thinner part
62a: first cushioning material
63: step face
64: thicker part
64a: second cushioning material
65: distal end face
66: extended portion
66a: third cushioning material
68a: extended portion
70: medium resin layer
100: catheter (first embodiment)
100a: catheter (second embodiment)
100b: catheter (third embodiment)
100c: catheter (fourth embodiment)
110: catheter body (fifth embodiment)
110a: catheter body (sixth embodiment)
110b: catheter body (seventh embodiment)
111A: distal end-side constant diameter part (fifth and sixth embodiments)
111B: proximal end-side constant diameter part (fifth and sixth embodiments)
111C: connection part (fifth and sixth embodiment)
112: catheter distal end part (fifth embodiment)
112a: catheter distal end part (sixth embodiment)
112b: catheter distal end part (seventh embodiment)
113A: first inner peripheral surface (fifth and sixth embodiments)
113B: second inner peripheral surface (fifth and sixth embodiments)
113C: third inner peripheral surface (fifth and sixth embodiments)
114: catheter proximal end part (fifth embodiment)
114a: catheter proximal end part (sixth embodiment)
114b: catheter proximal end part (seventh embodiment)
116: catheter connection part (fifth embodiment)
116a: catheter connection part (sixth embodiment)
116b: catheter connection part (seventh embodiment)
118: connector (fifth to seventh embodiments)
119: chip (fifth to seventh embodiments)
120: coil body (fifth embodiment)
120a: coil body (sixth embodiment)
120b: coil body (seventh embodiment)
122: wire (coil body) (fifth embodiment)
122a: wire (coil body) (sixth embodiment)
122b: wire (coil body) (seventh embodiment)
124: proximal end-side coil (fifth embodiment)
124a: proximal end-side coil (sixth embodiment)
124b: proximal end-side coil (seventh embodiment)
126: connection-side coil (fifth embodiment)
126a: connection-side coil (sixth embodiment)
126b: connection-side coil (seventh embodiment)
130: braided body (fifth and seventh embodiments)
130a: braided body (sixth embodiments)
130A: extracoil braided portion (fifth to seventh embodiments)
130B: intracoil braided portion (fifth to seventh embodiments)
131A: first constant diameter part (fifth and sixth embodiments)
131B: second constant diameter part (fifth and sixth embodiments)
131C: reduced diameter part (fifth and sixth embodiments)
132: wire (braided body) (fifth and seventh embodiments)
132a: wire (braided body) (sixth embodiment)
134A: distal end-side braided body (sixth embodiment)
134B: proximal end-side braided body (sixth embodiment)
134C: connection-side braided body (sixth embodiment)
140: inner resin layer (fifth to seventh embodiments)
150: outer resin layer (fifth to seventh embodiments)
200: catheter (fifth embodiment)
200a: catheter (sixth embodiment)
200b: catheter (seventh embodiment)
D1: first outer diameter
D2: second outer diameter
D3: third outer diameter
E1: first inner diameter
E2: second inner diameter
L1: first thickness
L2: second thickness

## Claims

1. A catheter comprising:
a coil body;
a braided body formed into a tubular shape by braiding a plurality of wires, the braided body having an intracoil braided portion arranged on an inner peripheral side of the coil body and an extracoil braided portion arranged so as to extend beyond the coil body toward a distal end;
an inner resin layer coating an inner peripheral surface of the braided body;
a cushioning material in contact with a part closer to the coil body in the extracoil braided portion of the braided body as well as in contact with a distal end of the coil body, the cushioning material having rigidity between rigidity of the braided body and rigidity of the coil body; and
an outer resin layer coating an outer peripheral surface of the extracoil braided portion of the braided body, the cushioning material, and the coil body.

2. The catheter according to claim 1,
wherein the extracoil braided portion of the braided body has a reduced diameter part having an outer diameter reduced toward a distal end of the catheter, and a constant diameter part adjacent to a distal end of the reduced diameter part and having the same outer diameter throughout an entire length in an axial direction of the catheter.

3. The catheter according to claim 1 or claim 2,
wherein an inner diameter of the inner resin layer in at least a part of the extracoil braided portion is smaller than the inner diameter of the inner resin layer in the intracoil braided portion.

4. The catheter according to any one of claim 1 to claim 3,
wherein a radial length of the catheter in the vicinity of a distal end of the cushioning material is smaller than the radial length in the vicinity of a proximal end of the cushioning material.

5. The catheter according to any one of claim 1 to claim 4,
wherein the cushioning material has an extended portion extending so as to lie over a plurality of wires, the plurality of wires composing the coil body as well as being adjacent to each other.

6. The catheter according to any one of claim 1 to claim 5,
comprising a medium resin layer filled in gaps in the braided body and is disposed between the outer resin layers and the inner resin layers.

7. A catheter comprising:
a coil body;
a braided body formed into a tubular shape by braiding a plurality of wires, the braided body having an intracoil braided portion arranged on an inner peripheral side of the coil body and an extracoil braided portion arranged so as to extend beyond the coil body toward a distal end;
an inner resin layer coating an inner peripheral surface of the braided body; and
an outer resin layer coating an outer peripheral surface of the extracoil braided portion of the braided body and the coil body;
wherein the catheter has a catheter distal end part including the extracoil braided portion, a catheter proximal end part including the coil body and the intracoil braided portion, and a catheter connection part connecting the catheter distal end part and the catheter proximal end part in an axial direction of the catheter;
wherein the catheter distal end part has a flexural rigidity of 0.005 gf·cm²/cm or more to less than 0.05 gf·cm²/cm;
wherein the catheter connection part has a flexural rigidity of 0.05 gf·cm²/cm or more to less than 1.4 gf·cm²/cm; and
wherein the catheter proximal end part has a flexural rigidity of 1.4 gf·cm²/cm or more to less than 3.0 gf·cm²/cm.

8. The catheter according to claim 7,
wherein a part of the coil body is placed in the catheter connection part, and
wherein the wire composing the part of the coil body becomes thinner toward a distal end of the catheter connection part.

9. The catheter according to claim 7 or claim 8,
wherein a part of the extracoil braided portion is placed in the catheter connection part, and
wherein the wire composing the part of the extracoil braided portion has a diameter becoming thicker toward a distal end of the coil body.

10. The catheter according to any one of claim 7 to claim 9,
wherein an outer diameter of the catheter is the same throughout an entire length.
